(19) Europäisches Patentamt

European Patent Office

Office européen des brevets

(11) EP 0 835 940 A1

(12) EUROPEAN PATENT APPLICATION

(43) Date of publication:
15.04.1998 Bulletin 1998/16

(21) Application number: 97117365.3

(22) Date of filing: 08.10.1997

(51) Int. Cl.⁶: $C12Q\ 1/68$, $C12Q\ 1/02$, $C12N\ 15/74$, $C12N\ 1/20$ // $C02F3/34$

(84) Designated Contracting States:
AT BE CH DE DK ES FI FR GB GR IE IT LI LU MC NL PT SE

(30) Priority: 08.10.1996 JP 267053/96

(71) Applicant:
KURITA WATER INDUSTRIES LTD.
Shinjuku-ku Tokyo (JP)

(72) Inventor: Iizumi, Taro
Shinjuku-ku, Tokyo (JP)

(74) Representative: Merten, Fritz
Patentanwalt
Tristanstrasse 5
90461 Nürnberg (DE)

(54) **Method for measurement of inhibition of nitrification activity by ammonia-oxidizing bacteria**

(57) Ammonia-oxidizing bacteria are transformed by introduction of a luciferase gene downstream from and in-frame with a promoter region that is activated under conditions wherein nitrification occurs in ammonia-oxidizing bacteria. In the presence of an inhibitor of nitrification activity, the activity of the products of the luciferase gene is diminished, leading to a decreased luminescence. The degree of luminescence provides a measure of the level of nitrification activity in the culture.

EP 0 835 940 A1

## Description

### BACKGROUND OF THE INVENTION

The present invention relates to a method for measuring inhibition of nitrification activity in ammonia-oxidizing bacteria. In particular, the present invention relates to a method for measuring inhibition of nitrification activity in ammonia-oxidizing bacteria which employs recombinant ammonia-oxidizing bacteria transformed by a plasmid bearing a luciferase gene.

Ammonia-oxidizing bacteria, such as the *Nitrosomonas* spp., are autotrophic bacteria which produce energy by oxidizing ammonia to produce nitrous acid, according to the following equation (1):

$$2NH_3 + 3O_2 \rightarrow 2HNO_2 + 2H_2O \tag{1}$$

The ammonia-oxidizing bacteria are widely distributed in the environment in soil, water, waste water plants and the like. The ammonia oxidation reaction catalyzed by these bacteria plays an important role in nitrogen cycling in the environment.

The process for removal of ammonia nitrogen from sewage water mimics the natural nitrogen cycle. Ammonia in sewage water is oxidized to nitrous acid ($NO_2$) or nitric acid ($HNO_3$) by ammonia-oxidizing bacteria and nitrite-oxidizing bacteria. The nitric acid or nitrous acid is reduced to nitrogen gas by bacteria which are capable of nitrogen respiration. Nitrogen gas is thereby released into the air. However, often this nitrogen removal process is unstable, compared to the conventional BOD removal method. When this happens, the processing capacity is lowered, and there may also be a decline in the quality of the treated water.

The main reason for the instability of the autotrophic bacterial nitrification process is that autotrophic ammonia-oxidizing bacteria in activated sludge are more sensitive to environmental changes than heterotrophic microorganisms. Ammonia-oxidizing activity in these bacteria is particularly sensitive to environmental changes, such as temperature shifts or pH shifts, and to the presence of certain chemical substances at low concentrations in the waste water. For example, it is known that ammonia monooxygenase, which catalyzes the initial oxidation reaction in the ammonia oxidation pathway, is easily inactivated by various chemical substances or by environmental changes.

For the operational maintenance of a waste water treatment plant which employs autotroptic ammonia-oxidizing microorganisms, it is unrealistic to attempt to identify and quantitate the cause or causes of reduced ammonia-oxidizing activity by instrumental analysis or chemical analysis, due to the wide range of inhibitory substances and environment conditions which influence the activity. As a result, there is a need for a measurement method which can evaluate the overall ammonia-oxidizing activity of the microorganisms and detect any inhibitory effect. A method which can quickly and easily detect a reduction in the nitrification activity of ammonia-oxidizing bacteria is highly desirable. This method should preferably produce measurement results which can be rapidly applied in the operational maintenance of a waste water treatment plant. This method should also preferably not require any special biochemical training for application by a waste water treatment plant worker. Furthermore, this method should also preferably be convenient and rapid.

In the prior art, measurement of the rate of ammonia oxidation under simulated environmental conditions has been used to detect inhibition of ammonia-oxidizing activity Ammonia-oxidizing bacteria, or a mixed population of organisms in activated sludge which include ammonia-oxidizing bacteria, are suspended in an ammonia-containing reagent. The reaction mixture is then incubated aerobically under conditions which simulate the environmental conditions. Ammonia oxidation rates are measured as changes in ammonia concentration, dissolved oxygen concentration, or nitrous acid concentration. Reductions in the rate of ammonia oxidation can thereby be detected.

However, with these methods, the measurement of the ammonia oxidation rates must be made regularly. The incubations or cultures may have to be continued for long periods of time. Furthermore, measurement of the index substances may require several time-consuming and complicated steps, and may have to be performed by highly skilled personnel. As a result, these methods lack ease and rapidity.

### OBJECTS AND SUMMARY OF THE INVENTION

It is accordingly an object of the present invention to overcome the limitations of the prior art, including those discussed above.

It is another object of the present invention to provide a means to monitor nitrification activity in waste water treatment facilities which utilize ammonia-oxidizing bacteria.

It is another object of the present invention to provide a means to detect inhibition of nitrification activity in ammonia-oxidizing bacteria.

It is another object of the present invention to provide a means to detect inhibition of nitrification activity in ammonia-oxidizing bacteria that is easily and rapidly applied.

Briefly stated, ammonia-oxidizing bacteria are transformed by introduction of a luciferase gene downstream from and in-frame with a promoter region that is activated under conditions wherein nitrification occurs in ammonia-oxidizing bacteria. In the presence of an inhibitor of nitrification activity, the activity of the products of the luciferase gene is diminished, leading to a decreased luminescence. The degree of luminescence provides a measure of the level of nitrification activity in the culture.

According to an embodiment of the present invention, a method for detecting inhibition of nitrifying activity in ammonia-oxidizing bacteria, comprises the steps of incubating a sample solution with a recombinant ammonia-oxidizing bacteria, the recombinant ammonia-oxidizing bacteria having a DNA construct, the DNA construct bearing a gene sequence encoding luciferase, the gene sequence being located downstream from and in-frame with a promoter region, the promoter region capable of initiating expression of the gene sequence under conditions wherein nitrification occurs in the ammonia-oxidizing bacteria, and measuring a luminescent response of the ammonia-oxidizing bacteria, whereby the luminescent response indicates a degree of inhibition of the nitrifying activity.

According to another embodiment of the present invention, a recombinant bacterium comprises an ammonia-oxidizing bacterium having a luciferase gene downstream from and in-frame with a promoter region, the promoter region capable of initiating expression of the luciferase gene under conditions wherein nitrification occurs in the ammonia-oxidizing bacteria

The above, and other objects, features and advantages of the present invention will become apparent from the following description read in conjunction with the accompanying drawings, in which like reference numerals designate the same elements.

## BRIEF DESCRIPTION OF THE DRAWINGS

Figure 1 is a schematic drawing showing the construction of plasmids pALUX10 or pHLUX20 of the present invention.

Figure 2 is a schematic drawing which shows the construction of plasmids pKTK40 of the present invention.

Figure 3 is a graph which shows the results of the luminescent response of *N. europaea* (pALUX10) to nitrification inhibiting substances.

Figure 4 is a graph which shows the results of the luminescent response of *N. europaea* (pHLUX20) to nitrification inhibiting substances.

## DETAILED DESCRIPTION OF THE PREFERRED EMBODIMENT

The ammonia-oxidizing bacteria, which are preferably used as the host for the present invention, oxidize ammonia and thereby generate nitric acid. They are autotrophic bacteria which, by using the energy obtained through this process, assimilate carbon dioxide. The ammonia-oxidizing bacteria include *Nitrosomonas* spp., *Nitrosospira* spp., and *Nitrosovibrio* spp. In particular, the present invention contemplates the use of *Nitrosomonas europaea* IFO14298. These ammonia-oxidizing bacteria can be obtained through public microbiological storage facilities, such as ATCC (American Type Culture Collection) or the IFO (Institute for Fermentation, Osaka). *Nitrosomonas* bacteria specifically isolated for denitrification processing of biological sludge can be used as well.

Ammonia-oxidizing bacteria obtain all of the energy required for growth from the oxidation of ammonia ($NH_3$). The series of oxidation reactions which begins with ammonia oxidation is catalyzed by ammonia monooxygenase and hydroxylamine oxidoreductase. Ammonia monooxygenase catalyzes the oxidation of ammonia to hydroxylamine, as shown in the following reaction equation (2):

$$NH_3 + O_2 + 2H^+ + 2e^- \rightarrow NH_2OH + H_2O \qquad (2)$$

Hydroxylamine oxidoreductase then catalyzes the oxidation of hydroxylamine to nitrous acid, as shown in the following reaction equation (3):

$$NH_2OH + H_2O \rightarrow NO_2^- + 5H^+ + 4e^- \qquad (3)$$

The latter reaction is the energy generating reaction. For one molecule of ammonia oxidized, four electrons are generated. The electrons are first delivered to cytochrome $C_{554}$, then to a still unidentified electron acceptor. Of these electrons, approximately half are brought to an electron transport system and used for ATP generation. The other half are used to create the reducing power needed in the oxidation reaction catalyzed by ammonia monooxygenase.

These nitrifying enzymes play a central role in the metabolism of ammonia-oxidizing bacteria. Ammonia-oxidizing activity in these bacteria is particularly sensitive to environmental changes, such as temperature or pH, and to the presence of certain chemical compounds at low concentrations. In particular, ammonia monooxygenase is highly sensitive to environmental changes, and the inhibition of ammonia monooxygenase activity is a major factor in the inhibition of nitrification.

Luciferases are luminescent enzymes which cause the phenomenon known as bioluminescence. These can be broadly divided into those of animal cell origin, such as luciferases from fireflies and the like, and those of bioluminescent bacterial origin, such as *Vibrio* spp. and the like. The luciferases of animal cell origin emit light by catalyzing the oxidation reaction shown in reaction equation (4):

$$\text{Luciferin} + O_2 + \text{ATP} \rightarrow \text{oxyluciferin} + CO_2 + \text{ADP} + H_4P_2O_7 + \lambda \tag{4}$$

$\lambda$ = emitted visible light

The luciferases of bacterial origin emit light by catalyzing the oxidation reaction shown in reaction equation (5):

$$\text{FMNH}_2 + \text{RCHO} + O_2 \rightarrow \text{FMN} + \text{RCOOH} + H_2O + \lambda \tag{5}$$

In the bacterial luciferase reaction, $\text{FMNH}_2$ is generated from NADH or NADPH by a reaction catalyzed by FMN reduction enzyme. When there is a constant quantity of luciferase and FMN reduction enzyme, the strength of luminescence depends on the concentration of NADH and NADPH. In the ammonia-oxidizing bacteria, NADH is created by using approximately 1/40 of the total reducing power generated by hydroxylamine oxidoreductase. Therefore, when ammonia monooxygenase and/or hydroxylamine oxidoreductase are inhibited by a nitrification inhibitory substance, the series of oxidation reaction which begins with ammonia oxidation is terminated, leading to a dramatic decrease in NADH concentration. As a result, by monitoring the intracellular NADH concentration, the degree of nitrification inhibition can be measured. The intracellular NADH concentration can be easily monitored by use of a bacterial luciferase reaction.

With regard to the use of animal cell luciferase in place of bacterial luciferase, when the ammonia oxidation enzymes are inhibited by nitrification inhibition substances, the generation of reductant is terminated, leading to a dramatic decline in ATP concentration. As a result, by monitoring intercellular ATP concentration, the strength of the inhibition of nitrification can be measured. The intercellular ATP concentration can be easily monitored, using an animal cell luciferase.

The present invention uses the luciferase reactions described above to monitor the intracellular levels of NADH and/or ATP and thereby determine the level of ammonia oxidizing activity in ammonia-oxidizing bacteria. Ammonia-oxidizing bacteria are transformed with a plasmid bearing the genes required to catalyze the luciferase reaction. When these genes are expressed in the ammonia-oxidizing bacteria, the intercellular levels of NADH and/or ATP can be periodically or continuously monitored.

As stated above, luciferase from various biological sources, such as animal cells or bioluminescent bacteria, can be used in the present invention. However, because it is easier to culture the bioluminescent bacteria, and because luciferase from animal cells requires a substrate (luciferin) not found in normal bacterial cells, it is preferable to use bacterial luciferase genes from *Vibrio* or *Photobacterium* or the like. More particularly, bacterial luciferase genes useful in the present invention include those in the *lux* gene group, such as *luxAB* or DNA fragments which include these gene groups. The nucleotide sequences of a number of luciferase genes of bacterial origin are known. These genes include: *luxAB* genes from *P. leiognathi* PL741 (reference: *J. Biolumin. Chemilumin.*, 4:326-341 (1989)) and the *luxAB* genes from *V. harveyi* ATCC33843 (reference: *J. Biol. Chem.*, 260:6139-6146 (1985), *J. Biol. Chem.*, 261:4805-4811 (1986)). Luciferase genes from strains of *Vibrio*, *Photobacterium*, or the like which have been isolated from the environment may be used, but microorganisms obtained from public microorganism storage facilities such as ATCC, IFO, and the like are preferable.

The luciferase genes are cloned from DNA extracted and purified from the above biological cells by well-known methods. For example, a gene library is created, using host-vector systems which are easily manipulated and well characterized. Examples of such hosts include *E. coli* or the like; examples of such vectors include plasmids or the like. The isolated DNA can be probed using a DNA probe which corresponds to the amino acid sequence of a luciferase protein or the nucleotide sequence of a luciferase gene. Screening for transformants may be performed by monitoring the phenotype of the transformed cells. Even more conveniently, by using the DNA sequence corresponding to the amino acid sequence of the target luciferase protein, or the DNA sequence corresponding to an amino acid region highly conserved among luciferases from different species of bacteria, cloning can be conducted using PCR (Polymerase Chain Reaction).

A promoter region is necessary upstream of the luciferase gene to express the luciferase gene within ammonia-oxidizing bacteria. A transcription initiation region which the RNA polymerase of the ammonia-oxidizing bacteria can recognize must be included within the promoter region. The RNA polymerase recognition sequence of the ammonia-oxidizing bacteria has not been reported previously. As a result, it is preferable to use promoters from known genes of ammonia-oxidizing bacteria and which clearly have the configuration and function of a promoter. The upstream region on the 5' side of a known gene where the presence of a promoter is hypothesized may also be used. In particular, the upstream region on the 5' side of the ammonia monooxygenase gene (*amo*) from *N. europaea* (reference: *J. Bacteriol.* 175:2436-2444 (1993)), the promoter region of the hydroxylamine oxidoreductase gene (*hao*) from *N. europaea* (reference: *J. Bacteriol.* 176:3148-3153 (1994)), and DNA fragments which include these promoter regions or 5' upstream regions may be used. In addition, promoter regions from other organisms may be used, as long as these promoters function in ammonia-oxidizing bacteria.

By introducing a fusion gene linking the luciferase gene downstream of this promoter region into the host ammonia-oxidizing bacteria, the desired recombinant ammonia-oxidizing bacteria is obtained. The fusion gene is obtained by linking the promoter region to the luciferase gene by any appropriate method. The introduced fusion gene may exist within the host as a satellite DNA, by using a vector such as a plasmid or a phage or the like which can independently replicate within the host ammonia-oxidizing bacteria. The target fusion gene can also be introduced into the host chromosome by integration through transposons, homologous recombination, and the like.

The recombinant ammonia-oxidizing bacteria can then be cultured by well-known methods. The culturing conditions should be appropriate for the recombinant bacteria. For example, the temperature should be between 20-30 °C, and the nutrient culture medium should be one generally used for ammonia-oxidizing bacteria, such as P culture medium. The composition of P medium is described below. The culture medium may be liquid or solid culture medium, but liquid culture medium is preferable. When using a recombinant which contains a plasmid bearing an antibiotic resistance marker, it is preferable to add an appropriate amount of antibiotic needed to stabilize the plasmid. Normally, an exponential growth phase is reached within about 3-7 days of culture. For example, during exponential growth in P medium, the bacterial cell concentration reaches $1 \times 10^6$-$1 \times 10^7$ cells/ml. The nitrous acid concentration in the same culture solution reaches 1-10 mM.

In order to measure inhibition of nitrification using the recombinant ammonia-oxidizing bacteria, a reagent which can determine the presence or absence of nitrification inhibition is added to the above recombinant culture solution. After further culturing, the strength of luminescence demonstrated by the recombinant ammonia-oxidizing bacteria can be measured. The culture solution is preferably in an exponential growth phase, and preferably at around 20-30 °C. The bacteria or the culture solution which is in exponential growth stage can be immediately assayed, or, alternatively, stored by freezing or by lyophilization. The cells or solutions which have been stored in low temperatures or as freeze-dried samples can be restored and used without difficulty. After adding the reagent, the cells are further cultured for a period of time sufficient to observe a change in luminescent strength. Culturing for a period of time between around 1 minute to 1 hour is usually appropriate.

In a positive control experiment where there is no inhibition of nitrification activity, there is a high concentration of NADH or ATP within the cell, and therefore there is a relatively strong luminescence. In contrast, if there is inhibition of nitrification activity of the ammonia-oxidizing bacteria, the intracellular concentration of NADH or ATP is inadequate for luminescence. As a result, a relatively weak luminescence is generated, or there may be no luminescence at all. Luminescence can be quantitatively measured using a measuring device such as a luminometer.

The degree of decline in luminescence is proportional to the degree of inhibition of nitrification. Therefore, by measuring the presence or absence of luminescence or a decrease in luminescence strength, the presence or absence of inhibition and the degree of inhibition of nitrification activity can be evaluated.

Because the reaction occurs in a short time, the measuring time is short. Because the luminescence can be measured without destroying the recombinant cells, the operation is convenient and rapid. Automated monitoring is also possible.

It is preferable to measure the luminescence under conditions where the activity of luciferase and the biological activity of ammonia-oxidizing bacteria can be maintained at a high level. The conditions preferably include a normal temperature of 20-30 ° C, at a pH of 7.5-8.0.

When a substrate for luciferase is necessary, for example a long-chain aldehyde or the like, the substrate must be added for the luminescence reaction to proceed. Examples of the added substrate include long-chain aldehydes such as n-decylaldehyde, n-nonylaldehyde or the like. With recombinants which contain genes which contribute to the production of substrate, no addition of substrate is necessary. These genes include, for example, the aldehyde synthesis genes of *Vibrio, Photobacterium*, or the like. In particular, the aldehyde synthesis genes include the *luxCDE* gene of *Vibrio harveyi*.

There are no restrictions on the type of nitrification-inhibition substances which are detected by this method. For example, this method can detect nitrification-inhibiting substances such as allylthiourea, nitrapyrin (2-chloro-6-trichloromethyl pyridine), or mixtures of these, as well as unidentified or even previously unknown nitrification inhibition substances. Besides nitrification-inhibiting substances, the present method can measure nitrification inhibition caused by environmental changes, such as changes in pH, temperature, and the like.

The plasmids, microorganisms, and the like which are used in the embodiment of the present invention are as follows.

*Nitrosomonas europaea* IFO14298 - Listed in "List of Cultures 1992 Microorganisms 9th Edition", published by IFO.

*Vibrio harveyi* ATCC33843 - ATCC No. 33843 (Catalogue of Bacteria and Phages, 17th edition, 1991).

*Escherichia coli* HB101 - Purchased from Asian Spinning Corporation (Toyo Boseki Corp.) as a competent cell preparation.

pHSG296 - Plasmid vector for *E. coli*. Purchased from Takara Shuzo Corporation.

pKK223-3 - Plasmid vector for *E. coli*. Purchased from Pharmacia Biotech Corporation.

pKT240 - IncQ type plasmid. ATCC No. 37258 (Catalogue of Recombinant DNA Materials 2nd Edition, 1991).

**Construction of recombinant ammonia-oxidizing bacteria**

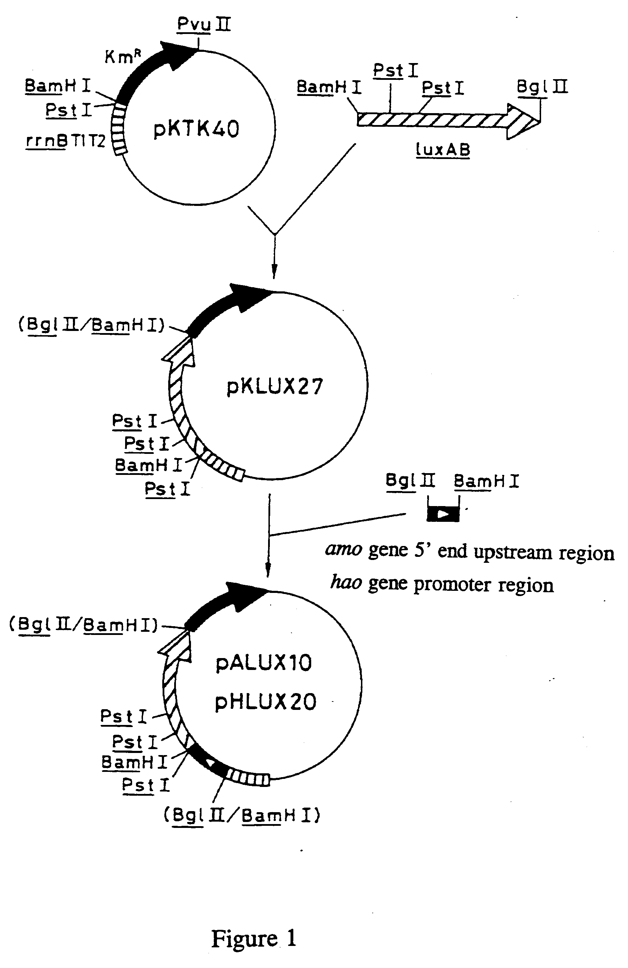

The construction of plasmids pALUX10 and pHLUX20, which encode the luciferase genes (*luxAB* gene) of *V. harveyi* ATCC33843 was performed as described below. In these plasmids, the *luxAB* genes are ligated downstream from a region which demonstrates some promoter activity in *N. europaea.* Figure 1 is a schematic diagram showing the construction of these plasmids. The plasmids were introduced into the ammonia-oxidizing bacteria as described below.

Cloning of *luxAB* genes of *V. harveyi*

From the reported *luxAB* gene sequence of *V. harveyi* ATCC33843, oligonucleotide primers LX117 and LX2268, which correspond to the 5'-flanking region and the 3'-flanking region of the *luxAB* gene sequence, respectively, were synthesized. The sequences of these oligonucleotides are shown in Table 1.

### Table 1. PCR primers for cloning *luxAB* gene

| LX117<br>SEQ ID 1 | 5'-CG<u>GGATCC</u>AACAAATAAGGAAATGTTATG-3'<br>*Bam*HI |
|---|---|
| LX2268<br>SEQ ID 2 | 5'-CC<u>AGATCT</u>TCCATATAAATGCCTCTATTAG-3'<br>*Bgl*II |

PCR was conducted using LX117 and LX2268 as primers and using *V. harveyi* ATCC33843 total chromosomal DNA as a template. Takara PCR amplification kit from Takara Shuzo Corporation was used for the PCR reagents. *V. harveyi* chromosomal DNA (0.5 micrograms), 20 pmol of each primer, 5 microliters of 10x PCR buffer, 5 microliters of 2.5 mM dNTP mixture, and 0.5 microliters of Takara Taq polymerase (5 U/microliter) were added to a Perkin-Elmer Micro Amp™ tube. The volume was then adjusted to 50 microliters with distilled water. The reaction mixture was transferred to a Perkin Elmer GeneAmp™ 2400 PCR System. After pre-processing at 94 °C for 30 seconds, twenty-five PCR cycles were performed. Each cycle consisted of 30 seconds at 94 °C, 1 minute at 55 °C, and 1 minute at 72 °C. An additional post-processing step was conducted for 10 minutes at 72 °C.

After phenol-chloroform extraction of the amplified PCR product, the PCR product was recovered by ethanol precipitation. A mixture solution of TE-saturated phenol:chloroform:isoamyl alcohol with a volume ratio of 25:24:1 was added to the completed reaction solution. After centrifuging at 10,000×g for 5 minutes, the supernatant was recovered. A volume of 3 M sodium acetate which corresponded to 1/10 the volume of the supernatant was added. After mixing well, a volume of ethanol which corresponded to 2.5 volumes was added. After mixing, the solution was incubated at -80 °C for 15 minutes. After centrifuging at 10,000×g for 10 minutes, the precipitated PCR product was washed with 70% ethanol, and then vacuum dried.

The PCR product was dissolved in TE buffer solution (10 mM Tris-Cl, pH 8.0, 1 mM EDTA). The PCR product then was loaded into a 1% agarose electrophoresis gel. After electrophoresis, the gel was transferred to an aqueous solution containing 10 microgram/ml ethidium bromide. The gel was stained at room temperature for 30 minutes, then thoroughly rinsed with water. The DNA bands were detected by UV-light illumination. A clear DNA band was detected at a position corresponding to 2.2 kb. The gel containing this DNA band was excised. The DNA contained in the gel was extracted using DNA CELL™ (Kusano Chemical Instrument Production). The 2.2 kb DNA fragment which was recovered contained the *luxAB* genes which encode *V. harveyi* luciferase. This DNA fragment was phenol-chloroform extracted and ethanol precipitated as above. The recovered DNA was dissolved in TE buffer, and used in plasmid construction as described below.

Cloning of the promoter regions of *N. europaea*

PCR cloning of the 5' end upstream region of the *amo* gene of *Nitrosomonas europaea*, which is hypothesized to have a promoter, and the promoter region of the *hao* gene of *N. europaea*, in which the function and configuration of the promoter has been reported, was conducted using the primers shown in Tables 2 and 3. As a result, a 0.24 kb PCR product of the *amo* gene 5' end upstream region and a 0.35 kb PCR product of the *hao* gene promoter region were obtained.

### Table 2. Primers for cloning 5' end upstream region of *N. europaea amo* gene

| | |
|---|---|
| AMOP-1 SEQ ID 3 | 5'CG<u>AGATCT</u>TACCCGTTATTCCAATCTG-3'<br>*Bgl*II |
| AMOP-2 SEQ ID 4 | 5'-CG<u>GGATCC</u>TTATAGTTTAAAAGTAAC-3'<br>*Bam*HI |

### Table 3. Primers for cloning the promoter region of *hao* gene from *N. europea*

| | |
|---|---|
| HAOP-1 SEQ ID 5 | 5'-CG<u>AGATCT</u>TCGAAATATTGATGAGCAGC-3'<br>*Bgl*II |
| HAOP-2 SEQ ID 6 | 5'-CG<u>GGATCC</u>GTAAATATGCGGGTCAG-3'<br>*Bam*HI |

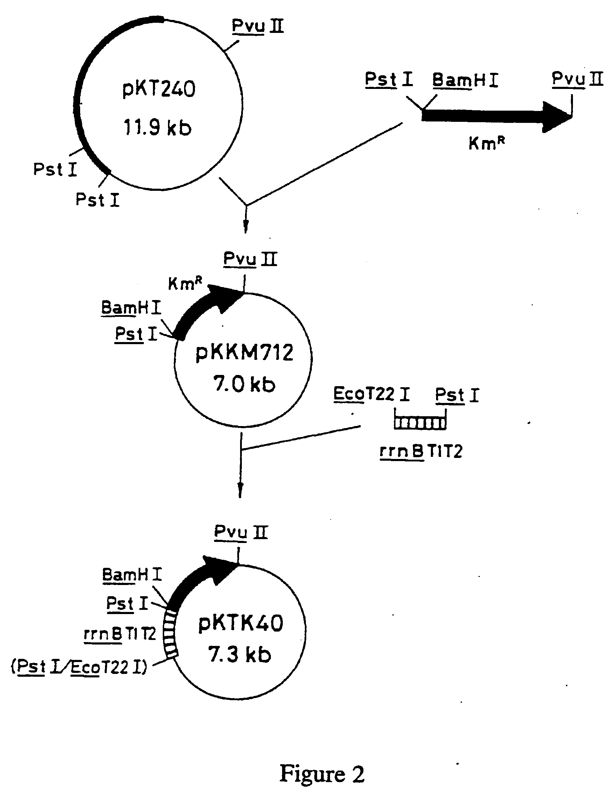

Plasmid pKTK40, shown in Figure 1, was constructed as shown in Figure 2. First, the kanamycin-acetyl-transferase gene (kanamycin-resistant gene, Km$^R$) of Tn903 was cloned from plasmid pHSG296, using the oligonucleotides shown in Table 4 as primers.

A 1.2 kb amplified product was obtained by the PCR reaction. This amplified product was digested with *Pst*I and *Pvu*II. The digest was ligated with a 5.8 kb fragment, previously obtained by digesting pKT240 with *Pst*I and *Pvu*II. The ligation product was transformed into *E. coli* HB101. Plasmid pKKM712 was obtained from the transformant (refer to Figure 2).

### Table 4. Primers for cloning kanamycin-acetyl-transferase gene.

| | |
|---|---|
| KAN-1 SEQ ID 7 | 5'-GG<u>CTGCAG</u>GATCCAGCCAGAAAGTGAGGG-3'<br>*Pst*I |
| KAN-2 SEQ ID 8 | 5'-GG<u>CAGCTG</u>CCGTCCCGTCAAGTCAGCG-3'<br>*Pvu*II |

Next, the terminator region of the *E. coli* 5S RNA gene (*rrnB*T1T2) was cloned from pKK223-3 plasmid DNA, using the oligonucleotides shown in Table 5 as primers.

**Table 5. Primers for cloning the terminator region of *E. coli* 5S RNA gene**

| 5ST-1 | 5'-GG<u>ATGCAT</u>GCGAGAGTAGGGAACTGCC-3' |
|---|---|
| **SEQ ID 9** | *Eco*T22I |
| | |
| 5ST-2 | 5'-GG<u>CTGCAG</u>CATGCGACAGGAAGAGTTTGTAGAAACGC-3' |
| **SEQ ID 10** | *Pst*I |

A 0.3 kb product was obtained by PCR. This product was digested by *Eco*T22I and *Pst*I, and ligated to pKKM712 which has been previously digested by *Pst*I and treated with alkaline phosphatase. The ligation product was transformed into *E. coli* HB101. Plasmid pKTK40 was obtained by the transformants (see Figure 2).

Plasmid construction and introduction into ammonia-oxidizing bacteria

Plasmids pALUX10 and pHLUX20 were constructed as shown in Fig. 1. The 2.2 kb PCR product containing *V. harveyi luxAB* was digested with *Bam*HI and *Bgl*II. The digest was ligated with pKTK40, which has been previously digested with *Bam*HI and treated with alkaline phosphatase. The ligation product was transformed into *E. coli* HB101. Plasmid pKLUX27 was obtained from the transformant (see Figure 1). Plasmid pKLUX27 was digested with *Bam*HI and treated with alkaline phosphatase, then ligated to a *Bam*HI and *Bgl*II digest of the PCR products encoding the *amo* gene 5' end upstream region or the promoter region of the *hao* gene. The ligation products were transformed into *E. coli* HB101. Plasmids pALUX10 (*amo-luxAB*) and pHLUX20 (*hao-luxAB*) were obtained from the transformant (see Figure 1). Plasmids pALUX10 and pHLUX20 contain the *lux*AB gene under transcriptional control of the *amo* gene 5' end upstream region, and the *hao* gene promoter, respectively.

Plasmids pALUX10 and pHLUX20 were introduced into N. *europaea* by electroporation. The mixture solution of pALUX10 or pHLUX20 and *N. europaea* cells was transferred to a chilled cuvette ( Bio-Rad, Richmond, CA, USA), with an electrode spacing of 0.2 cm. The mixture solution was immediately placed inside a Gene Pulsar cuvette chamber (Bio-Rad, Richmond, CA, USA) and electric pulses (5-12.5 kV/cm, 5.0 msec each) were applied. After the discharge, 1 ml of P medium was added directly to the cuvette. The suspension was transferred to a test tube and incubated in the dark at 30 °C for 3 days with shaking. The cells then were plated onto a solid P medium containing 1.2 % Gellan gum and 25 micrograms/l kanamycin, and incubated in the dark at 30 °C for 2 weeks. Recombinant N. *europaea* (pALUX10) and N. *europaea* (pHLUX20) were thereby obtained as kanamycin-resistant colonies.

**Measurement of nitrification inhibition using recombinant ammonia-oxidizing bacteria**

The recombinant ammonia-oxidizing bacteria N. *europaea* (pALUX10), obtained as above, was cultured in 100 ml of P culture medium (2.5 g/l $(NH_4)_2SO_4$, 0.7 g/l $KH_2PO_4$, 19.5 g/l $Na_2HPO_4$, 0.5 g/l $NaHCO_3$, 0.1 g/l $MgSO_4 \cdot 7H_2O$, 5 mg/l $CaCl_2 \cdot 2H_2O$, 1 mg/l Fe-EDTA, pH 8.0) containing 25 micrograms/l kanamycin, in a 500 ml flask. The culture was incubated with shaking under aerobic conditions in the dark at 30 °C until a nitrous acid concentration of approximately 5 mM was reached. 5 ml of culturing solution was transferred into each culturing test tube. Allylthiourea or nitrapyrin (2-chloro-6-trichloromethyl pyridine), which are typical inhibitors of ammonia monooxygenase, were added so that the final concentration was either 1 $\mu$M or 10 $\mu$M. The control lacked added inhibitors.

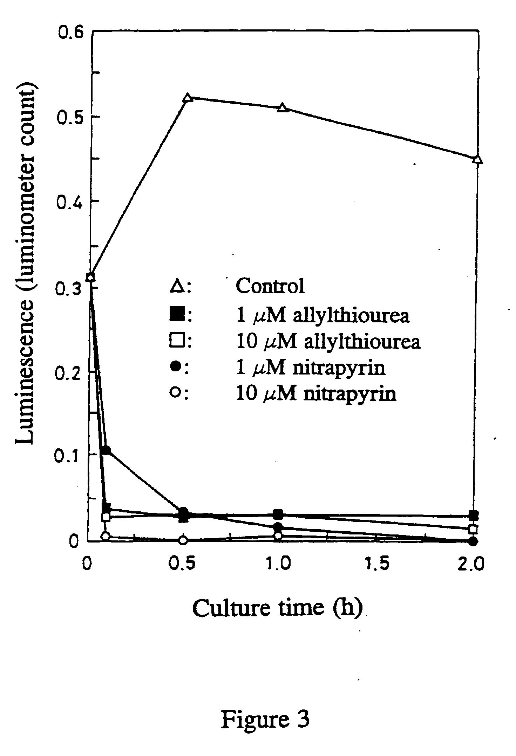

The shaking culture was continued at 30 °C. A portion of the culturing solution was periodically sampled, and its luminescence was measured. Luminescence measurement was performed by adding 0.2 ml of the culturing solution to a luminescence measuring cuvette having 10 microliters of n-decylaldehyde, which had been diluted tenfold with ethanol. For the measurement of luminescence, a Turner Design Company Luminometer Model 200e was used. Delay time was set at 5 seconds, and integrate time was set at 10 seconds. The results are shown in Figure 3.

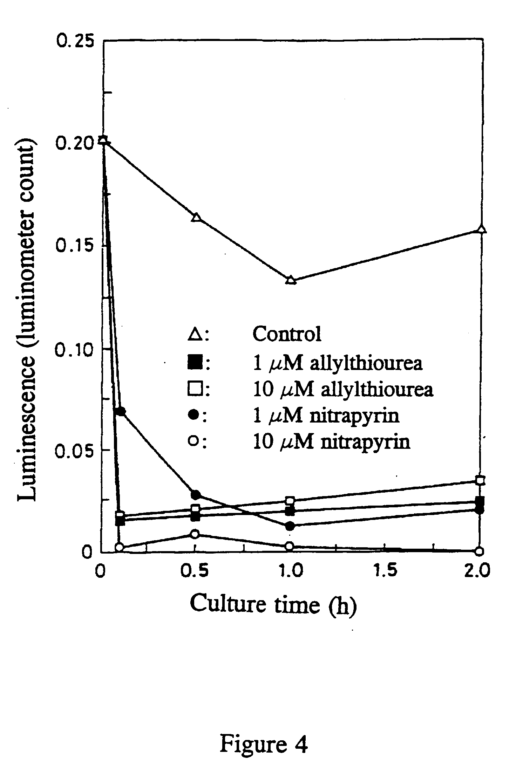

Nitrification inhibition in N. *europaea* (pHLUX20) was measured in the same manner as for N. *europaea* (pALUX10). The results of the luminescence measurement in N. *europaea* (pHLUX20) are shown in Figure 4.

Referring to Figs. 3 and 4, it is evident that addition of each of the ammonia monooxygenase inhibitors caused a rapid decline in intracellular NADH levels. Little difference was observed between 1 $\mu$M and 10 $\mu$M concentrations of the inhibitors, particularly after 30 minutes of incubation. In contrast, NADH levels in the control cultures remained high.

Therefore, the recombinant ammonia-oxidizing bacteria *N. europaea* (pALUX10) and *N. europaea* (pHLUX20) are effective to easily and rapidly measure changes in the rates of nitrification.

Having described preferred embodiments of the invention with reference to the accompanying drawings, it is to be understood that the invention is not limited to those precise embodiments, and that various changes and modifications may be effected therein by one skilled in the art without departing from the scope or spirit of the invention as defined in the appended claims.

## Claims

1. A method for detecting inhibition of nitrifying activity in ammonia-oxidizing bacteria, comprising the steps of:

   forming a recombinant ammonia-oxidizing bacteria;
   said recombinant ammonia-oxidizing bacteria having a DNA construct;
   said DNA construct bearing a gene sequence encoding luciferase;
   said gene sequence being located downstream from and in-frame with a promoter region;
   said promoter region capable of initiating expression of said gene sequence under conditions wherein nitrification occurs in said ammonia-oxidizing bacteria;
   incubating a sample solution with a culture of said recombinant ammonia-oxidizing bacterium; and
   measuring production of visible light catalyzed by said luciferase, whereby a degree of said production indicates a degree of inhibition of said nitrifying activity.

2. A method for detecting inhibition of nitrifying activity in ammonia-oxidizing bacteria according to claim 1, wherein said ammonia-oxidizing bacteria is one of a *Nitrosomonas* spp., a *Nitrosospira* spp., and a *Nitrosovibrio* spp.

3. A method for detecting inhibition of nitrifying activity in ammonia-oxidizing bacteria according to claim 1, wherein said ammonia-oxidizing bacteria is *Nitrosomonas europaea* IFO 14298.

4. A method for detecting inhibition of nitrifying activity in ammonia-oxidizing bacteria according to claim 1, wherein said gene sequence includes *luxAB* genes.

5. A method for detecting inhibition of nitrifying activity in ammonia-oxidizing bacteria according to claim 4, wherein said *luxAB* genes are derived from one of a strain of *Vibrio* spp. and *Photobacterium* spp.

6. A method for detecting inhibition of nitrifying activity in ammonia-oxidizing bacteria according to claim 4, wherein said *luxAB* genes are derived from *Vibrio harveyi* ATCC33843.

7. A method for detecting inhibition of nitrifying activity in ammonia-oxidizing bacteria according to claim 1, wherein said promoter region includes a promoter region for a gene encoding hydroxylamine oxidoreductase.

8. A method for detecting inhibition of nitrifying activity in ammonia-oxidizing bacteria according to claim 1, wherein said promoter region includes a promoter region for a gene encoding ammonia monooxygenase.

9. A method for detecting inhibition of nitrifying activity in ammonia-oxidizing bacteria according to claim 7, wherein said promoter region is from *Nitrosomonas europaea* IFO14298.

**10.** A method for detecting inhibition of nitrifying activity in ammonia-oxidizing bacteria according to claim 8, wherein said promoter region is isolated from *Nitrosomonas europaea* IFO14298.

**11.** A recombinant bacterium, comprising:

an ammonia-oxidizing bacterium having a luciferase gene downstream from, and in-frame with, a promoter region;
said promoter region capable of initiating expression of said luciferase gene under conditions wherein nitrification occurs in said ammonia-oxidizing bacteria.

**12.** A recombinant bacterium according to claim 11, wherein said ammonia-oxidizing bacteria is one of a *Nitrosomonas* spp., a *Nitrosospira* spp., and a *Nitrosovibrio* spp.

**13.** A recombinant bacterium according to claim 11, wherein said ammonia-oxidizing bacteria is *Nitrosomonas europaea* IFO 14298.

**14.** A recombinant bacterium according to claim 11, wherein said luciferase gene is a gene sequence which includes *luxAB* genes.

**15.** A recombinant bacterium according to claim 14, wherein said *luxAB* genes are derived from one of a strain of *Vibrio* spp. and *Photobacterium* spp.

**16.** A recombinant bacterium according to claim 14, wherein said *luxAB* genes are derived from *Vibrio harveyi* ATCC33843.

**17.** A recombinant bacterium according to claim 11, wherein said promoter region includes a promoter region for a gene encoding hydroxylamine oxidoreductase.

**18.** A recombinant bacterium according to claim 11, wherein said promoter region includes a promoter region for a gene encoding ammonia monooxygenase.

**19.** A recombinant bacterium according to claim 17, wherein said promoter region is from *Nitrosomonas europaea* IFO14298.

**20.** A recombinant bacterium according to claim 18, wherein said promoter region is from *Nitrosomonas europaea* IFO14298.

**21.** A recombinant bacterium according to claim 13, wherein said *luxAB* genes are derived from *Vibrio harveyi* ATCC33843, and said promoter region is from *Nitrosomonas europoea* IFO14298.

Figure 1

Figure 2

Figure 3

Figure 4

| | European Patent Office | EUROPEAN SEARCH REPORT | Application Number EP 97 11 7365 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | DE 44 31 964 A (BAYER AG)<br>* the whole document *<br>--- | 1-21 | C12Q1/68<br>C12Q1/02<br>C12N15/74 |
| Y | US 5 196 524 A (GUSTAFSON GARY D ET AL)<br>* the whole document *<br>--- | 1-21 | C12N1/20<br>//C02F3/34 |
| A | NL 7 906 900 A (AKZO NV)<br>* claim 1 *<br>--- | 1 | |
| A | DATABASE WPI<br>Week 9113<br>Derwent Publications Ltd., London, GB;<br>AN 91-093269<br>XP002053639<br>"Determining activity of ammoniacal nitrogen inhibitor "<br>& SU 1 581 743 A (VODOPYANOV V G) , 30 July 1990<br>* abstract *<br>--- | 1 | |
| A | WO 94 24286 A (ALLIED SIGNAL INC)<br>* the whole document *<br>----- | 1,7-21 | TECHNICAL FIELDS SEARCHED (Int.Cl.6)<br><br>C12Q<br>C12N |

The present search report has been drawn up for all claims

| Place of search | Date of completion of the search | Examiner |
|---|---|---|
| THE HAGUE | 28 January 1998 | Osborne, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

## SEQUENCE LISTING

### (1) GENERAL INFORMATION

(I) APPLICANT: Iizumi, Taro

(ii) TITLE OF INVENTION: METHOD FOR MEASUREMENT OF INHIBITION OF NITRIFICATION ACTIVITY BY AMMONIA-OXIDIZING BACTERIA

(iii) NUMBER OF SEQUENCES: 10

### (2) INFORMATION FOR SEQ ID NO. 1

(I) SEQUENCE CHARACTERISTICS

(A) LENGTH: 29

(B) TYPE: nucleic acid

(C) STRANDEDNESS: single

(D) TOPOLOGY: linear

(ii) MOLECULE TYPE: other nucleic acid - synthetic primer DNA

(iii) HYPOTHETICAL: no

(xi) SEQUENCE DESCRIPTION:SEQ ID NO:1:

CGGGATCCAA CAAATAAGGA AATGTTATG

### (2) INFORMATION FOR SEQ ID NO. 2

(I) SEQUENCE CHARACTERISTICS

(A) LENGTH: 30

(B) TYPE: nucleic acid

(C) STRANDEDNESS: single

(D) TOPOLOGY: linear

(ii) MOLECULE TYPE: other nucleic acid - synthetic primer DNA

(iii)   HYPOTHETICAL: no

(xi)   SEQUENCE DESCRIPTION:SEQ ID NO:2:

CCAGATCTTC CATATAAATG CCTCTATTAG

(2) INFORMATION FOR SEQ ID NO. 3

    (I)   SEQUENCE CHARACTERISTICS

        (A)   LENGTH: 27

        (B)   TYPE: nucleic acid

        (C)   STRANDEDNESS: single

        (D)   TOPOLOGY: linear

(ii)MOLECULE TYPE: other nucleic acid - synthetic primer DNA

(iii)   HYPOTHETICAL: no

(xi)   SEQUENCE DESCRIPTION:SEQ ID NO:3:

CGAGATCTTA CCCGTTATTC CAATCTG

(2) INFORMATION FOR SEQ ID NO. 4

    (I)   SEQUENCE CHARACTERISTICS

        (A)   LENGTH: 26

        (B)   TYPE: nucleic acid

        (C)   STRANDEDNESS: single

        (D)   TOPOLOGY: linear

(ii)MOLECULE TYPE: other nucleic acid - synthetic primer DNA

(iii)   HYPOTHETICAL: no

(xi)   SEQUENCE DESCRIPTION:SEQ ID NO:4:

CGGGATCCTT ATAGTTTAAA AGTAAC

(2) INFORMATION FOR SEQ ID NO. 5

    (I)   SEQUENCE CHARACTERISTICS

(A)    LENGTH: 28

(B)    TYPE: nucleic acid

(C)    STRANDEDNESS: single

(D)    TOPOLOGY: linear

(ii)MOLECULE TYPE: other nucleic acid - synthetic primer DNA

(iii)    HYPOTHETICAL: no

(xi)    SEQUENCE DESCRIPTION:SEQ ID NO:5:

CGAGATCTTC GAAATATTGA TGAGCAGC

(2) INFORMATION FOR SEQ ID NO. 6

(I)    SEQUENCE CHARACTERISTICS

(A)    LENGTH: 25

(B)    TYPE: nucleic acid

(C)    STRANDEDNESS: single

(D)    TOPOLOGY: linear

(ii)MOLECULE TYPE: other nucleic acid - synthetic primer DNA

(iii)    HYPOTHETICAL: no

(xi)    SEQUENCE DESCRIPTION:SEQ ID NO:6:

CGGGATCCGT AAATATGCGG GTCAG

(2) INFORMATION FOR SEQ ID NO. 7

(I)    SEQUENCE CHARACTERISTICS

(A)    LENGTH: 29

(B)    TYPE: nucleic acid

(C)    STRANDEDNESS: single

(D)    TOPOLOGY: linear

(ii)MOLECULE TYPE: other nucleic acid - synthetic primer DNA

(iii)    HYPOTHETICAL: no

(xi)    SEQUENCE DESCRIPTION:SEQ ID NO:7:

GGCTGCAGGA TCCAGCCAGA AAGTGAGGG

(2) INFORMATION FOR SEQ ID NO. 8

    (I)    SEQUENCE CHARACTERISTICS

        (A)    LENGTH: 27

        (B)    TYPE: nucleic acid

        (C)    STRANDEDNESS: single

        (D)    TOPOLOGY: linear

    (ii)MOLECULE TYPE: other nucleic acid - synthetic primer DNA

    (iii)    HYPOTHETICAL: no

    (xi)    SEQUENCE DESCRIPTION:SEQ ID NO:8:

GGCAGCTGCC GTCCCGTCAA GTCAGCG

(2) INFORMATION FOR SEQ ID NO. 9

    (I)    SEQUENCE CHARACTERISTICS

        (A)    LENGTH: 27

        (B)    TYPE: nucleic acid

        (C)    STRANDEDNESS: single

        (D)    TOPOLOGY: linear

    (ii)MOLECULE TYPE: other nucleic acid - synthetic primer DNA

    (iii)    HYPOTHETICAL: no

    (xi)    SEQUENCE DESCRIPTION:SEQ ID NO:9:

GGATGCATGC GAGAGTAGGG AACTGCC

(2) INFORMATION FOR SEQ ID NO. 10

    (I)    SEQUENCE CHARACTERISTICS

(A)    LENGTH: 37

(B)    TYPE: nucleic acid

(C)    STRANDEDNESS: single

(D)    TOPOLOGY: linear

(ii)MOLECULE TYPE: other nucleic acid - synthetic primer DNA

(iii)   HYPOTHETICAL: no

(xi)    SEQUENCE DESCRIPTION:SEQ ID NO:10:

GGCTGCAGCA TGCGACAGGA AGAGTTTGTA GAAACGC